# EUROPÄISCHE PATENTANMELDUNG

(11) **EP 0 963 739 A1**
(43) Veröffentlichungstag der Anmeldung: **15.12.1999**
(21) Anmeldenummer: 99110245.0
(22) Anmeldetag: 27.05.1999
(51) Int. Cl.: A61F 2/24

(54) **Herzklappenprothese**

(30) Priorität: 12.06.1998 DE 19826104
(71) Anmelder: Tricumed Medizintechnik GmbH, 24143 Kiel (DE)
(72) Erfinder: Otto, Karl-Heinz, 24146 Kiel (DE); Ebel, Thomas, Dr., 24147 Klausdorf/Schwentine (DE); Wieland, Manfred, 24146 Kiel (DE)
(74) Vertreter: Biehl, Christian, Dipl.-Phys.

(57) **Zusammenfassung**

Herzklappenprothese mit einem Klappenring (8) und wenigstens einer im wesentlichen gestreckt ausgebildeten, um eine in der Ebene des Klappenrings (8) liegende Achse verschwenkbar gelagerte Flügelklappe (1), bei der die Lager der Flügelklappen(n) jeweils durch einen am Klappenring (8) befestigten, die Schwenkachse definierenden Schwenkstift (2), eine in der Flügelklappe (1) ausgebildete, sich durch diese qoer zur Achse hindurch erstreckende Nut (3), deren Breite dem Durchmesser und deren Tiefe der Länge des Schwenkstifts (2) entspricht, einen am Klappenring (8) befestigten ersten Führungsstift (4), eine an der Flügelklappe (1) ausgebildete erste konvexe Gleitkurve (5), die mit dem ersten Führungsstift (4) zusammenwirkt, einen am Klappenring (8) befestigten zweiten Führungsstift (6), und eine an der Flügelklappe (1) ausgebildete zweite konvexe Gleitkurve (7), die mit dem zweiten Führungsstift (6) zusammenwirkt, wobei der über die gedachte Verbindungslinie zwischen den Mittelpunkten der Führungsstifte (4, 6) gemessene Abstand der beiden Gleitkurven (5, 7) voneinander in jeder Position der Flügelklappe dem lichten Abstand der beiden Führungsstifte (4, 6) voneinander entspricht, gebildet ist (bzw.sind).

## Beschreibung

Die Erfindung betrifft eine zum Ersatz von natürlichen Herzklappen dienende Herzklappenprothese mit einem Klappenring und wenigstens einer im wesentlichen gestreckt ausgebildeten, um eine in der Ebene des Klappenrings liegende Achse verschwenkbar an dem Klappenring gelagerte Flügelklappe.

Aus der DE 37 01 755 C1, der US 5 192 309, der US 4 328 592 und der DE 196 33 346 C1 ist es bekannt, bei Herzklappenprothesen die Lagerstellen der Flügelklappen so auszubilden, daß diese von dem Blutstrom "durchgespült" werden. Bei der DE 37 01 755 C1 erfolgt dies durch besondere Ein- und Auslauftrichter nach den Lagerstellen, bei der US 5 192 309 und der US 4 328 592 durch Längsnuten auf der Innenseite des Klappenrings. Bei der DE 196 33 346 C1 sind in Langlöcher in den Flügelklappen ragende Lagerzapfen vorgesehen.

Die WO 97 05 834 und die US 4 935 030 zeigen Gleitführungen für die Flügelklappen.

Der Erfindung liegt die Aufgabe zugrunde, eine Herzklappenprothese zu schaffen, deren in allen Bereichen vom Blut umströmtes Lager eine genaue Führung der Herzklappe gewährleistet.

Erfindungsgemäß wird diese Aufgabe durch die Merkmale des Anspruchs 1 gelöst, die Unteransprüche geben vorteilhafte Ausgestaltungen der Erfindung an.

Die vorgeschlagene Ausbildung der Lager mit einer den Schwenkstift aufnehmenden Nut erlaubt ein ständiges Ausspülen aller Bereiche des Lagers durch den Blutstrom, die Führungsstifte stellen im Zusammenwirken mit den Gleitkurven eine Zwangsführung der Klappe sicher.

Die Erfindung wird im folgenden anhand einer Zeichnung erläutert. Dabei zeigt:
- Fig. 1: das Lager in einer schematischen Abwicklung einer Draufsicht auf die Ringebene,
- Fig. 2: eine im Bereich des Lagers geschnitte Darstellung des Klappenflügels in der geschlossenen Position, und
- Fig. 3: eine im Bereich des Lagers geschnitte Darstellung des Klappenflügels in der geöffneten Position.

Die Herzklappenprothese besteht aus einem Klappenring 8 und wenigstens einer im wesentlichen gestreckt ausgebildeten, um eine in der Ebene des Klappenrings 8 liegende Achse verschwenkbar gelagerten Flügelklappe 1.

Die Flügelklappe 1 ist über jeweils zwei Lager an dem Klappenring 1 befestigt, von denen in den Figuren nur eines dargestellt ist.

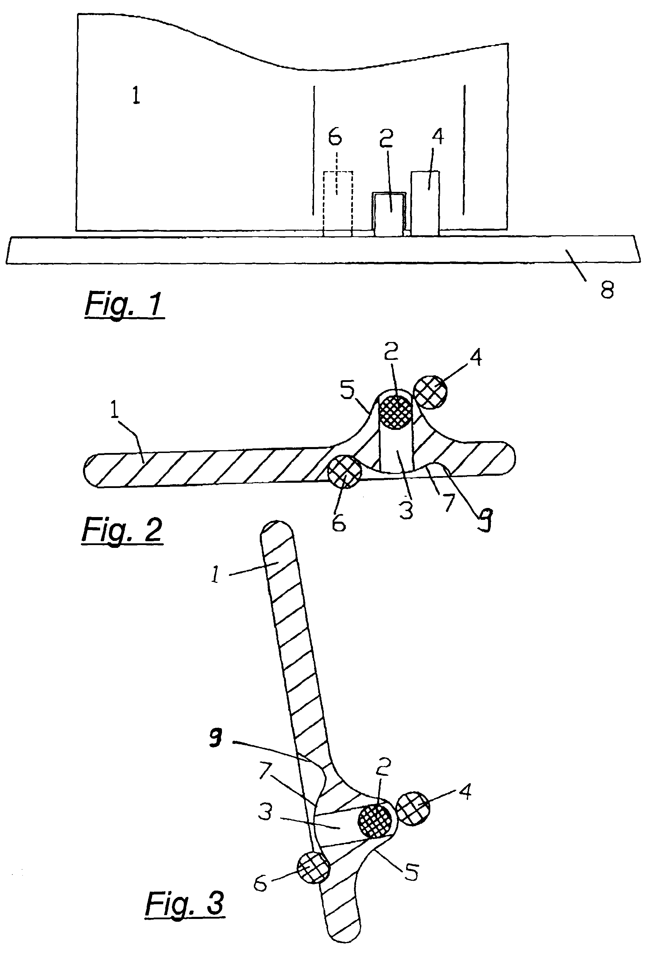

Das Lager besteht aus einem am in Figur 1 in einer Abwicklung dargestellten Klappenring 8 befestigten Schwenkstift 2, der gemeinsam mit dem entsprechenden Schwenkstift des anderen Lagers der jeweiligen Flügelklappe deren Schwenkachse definiert. In der Flügelklappe 1 ist eine sich durch diese hindurch quer zur Achse erstreckende Nut 3 ausgebildet, deren Breite dem Durchmesser und deren Tiefe der Länge des Schwenkstifts 2 entspricht.

Am Klappenring 8 ist ein erster Führungsstift 4 befestigt, der mit einer an der Flügelklappe 1 ausgebildeten ersten konvexen Gleitkurve 5 zusammenwirkt. Ein am Klappenring 8 befestigter zweiter Führungsstift 6 wirkt mit einer an der Flügelklappe 1 ausgebildeten zweiten konvexen Gleitkurve 7 zusammen.

Der lichte Abstand der beiden Führungsstifte 4, 6 voneinander entspricht dem über die Mitte des Schwenkstiftes 2 gemessenen Abstand der beiden Gleitkurven 5, 7 voneinander.

Die beiden Führungsstifte 4, 6 sind länger als der Schwenkstift 2.

An die konvexe zweite Gleitkurve 7 schließen sich konkave Anlageabschnitte 9 an, an denen die zwischen den Führungsstiften 4, 6 verlaufende Flügelklappe 1 in ihren in den Figuren 2 bzw. 3 gezeigten Endpositionen an dem zweiten Führungsstift 6 anliegt.

Zeichnerisch nicht dargestellt ist eine Ausbildung, bei der eine oder beide Führungsstifte 4, 6 ganz oder teilweise aus einem elastischen Material gefertigt sind. Diese Ausgestaltung hat den Vorteil, daß der Anschlag der Flügelklappen an die Stifte 4, 6 gedämpft wird. Eine teilweise oder vollständige Ausbildung der Führungsstifte aus einem Elastomer kann weiter dazu genutzt werden, die Flügelklappe in ihren Endpositionen in Richtung auf die nachfolgende Bewegung vorzuspannen, was sich günstig auf die Schnelligkeit des Schließ- bzw. Öffnungsverhalten auswirkt.

Es versteht sich, daß die Dimensionierung der einzelnen zusammenwirkenden Elemente so gewählt ist, daß ein ausreichend großes Spiel zwischen diesen gegeben ist.

## Patentansprüche

1. Herzklappenprothese mit einem Klappenring (8) und wenigstens einer im wesentlichen gestreckt ausgebildeten, um eine in der Ebene des Klappenrings (8) liegende Achse verschwenkbar gelagerte Flügelklappe (1),
dadurch gekennzeichnet, daß
die Lager der Flügelklappen(n) jeweils durch
- einen am Klappenring (8) befestigten, die Schwenkachse definierenden Schwenkstift (2),
- eine in der Flügelklappe (1) ausgebildete, sich durch diese qoer zur Achse hindurch erstreckende Nut (3), deren Breite dem Durchmesser und deren Tiefe der Länge des Schwenkstifts (2) entspricht,
- einen am Klappenring (8) befestigten ersten Führungsstift (4),
- eine an der Flügelklappe (1) ausgebildete erste konvexe Gleitkurve (5), die mit dem ersten Führungsstift (4) zusammenwirkt,
- einen am Klappenring (8) befestigten zweiten Führungsstift (6), und
- eine an der Flügelklappe (1) ausgebildete zweite konvexe Gleitkurve (7), die mit dem zweiten Führungsstift (6) zusammenwirkt,
- wobei der über die gedachte Verbindungslinie zwischen den Mittelpunkten der Führungsstifte (4, 6) gemessene Abstand der beiden Gleitkurven (5, 7) voneinander in jeder Position der Flügelklappe dem lichten Abstand der beiden Führungsstifte (4, 6) voneinander entspricht,
gebildet ist (bzw.sind).

2. Herzklappenprothese nach Anspruch 1, dadurch gekennzeichnet, daß die beiden Führungsstifte (4, 6) länger als der Schwenkstift (2) sind.

3. Herzklappenprothese nach Anspruch 1 oder 2, gekennzeichnet durch sich beidseitig an die konvexe zweite Gleitkurve (7) anschließende konkave Anlageabschnitte.

4. Herzklappenprothese nach einem der vorangehenden Ansprüche, dadurch gekennzeichnet, daß der erste Führungsstift (4) ganz oder teilweise aus einem elastischen Material gefertigt ist.

5. Herzklappenprothese nach einem der vorangehenden Ansprüche, dadurch gekennzeichnet, daß der zweite Führungsstift (7) ganz oder teilweise aus einem elastischen Material gefertigt ist.
